# EUROPEAN PATENT APPLICATION

(11) **EP 1 547 650 A1**
(43) Date of publication of application: **29.06.2005**
(21) Application number: 03447279.5
(22) Date of filing: 02.12.2003
(51) Int. Cl.: A61P 25/24, A61K 31/343, A61K 31/4545, G01N 33/48

(54) **Use of D4 and 5-HT2A antagonists, inverse agonists or partial agonists**

(71) Applicant: B & B Beheer NV, 3570 Alken (BE)
(72) Inventor: Buntinx, Erik, 3570 Alken (BE)
(74) Representative: De Clercq, Ann

(57) **Abstract**

The present invention relates to the use of compounds and compositions of compounds having D4 and/or 5-HT2A antagonistic, partial agonistic or inverse agonistic activity for the treatment of the underlying dysregulation of the emotional functionality of mental disorders (i.e. affect instability - hypersensitivity - hyperaesthesia - dissociative phenomena -...). The invention also relates to methods comprising administering to a patient diagnosed as having a neuropsychiatric disorder a pharmaceutical composition containing (i) compounds having D4 antagonistic, partial agonistic or inverse agonistic activity and/or (ii) compounds having 5-HT2A antagonistic, partial agonistic or inverse agonistic, and/or (iii) any known medicinal compound and compositions of said compounds. The combined D4 and 5-HT2A antagonistic, partial agonistic or inverse agonistic effects may reside within the same chemical or biological compound or in two different chemical and/or biological compounds.

## Description

### Field of the invention

The invention relates to the field of neuropsychiatry. More specifically, the invention relates to the use of compounds which have D4 and/or 5-HT2A antagonist, inverse agonist or partial agonist activity for the preparation of medicaments.

### Background of the invention

Clinical or real effectiveness of psychopharma is very rare via common pooping-out, many treatment-refractory patients and up to half of patients fail to attain remission (S. M. Stahl, Essential Psychopharmacology, 2000). Implications of not attaining remission for Mental Disorders are increased relapse rates, continuing functional impairment and increased suicide rate.

Clinical causes of not attaining remission by the Current Psychopharmacological Compounds are inadequate early treatment, underlying dysregulation of the emotional functionality (affect instability - hypersensitivity - hyperaesthesia - dissociative phenomena...) and competitive antagonism.

Dysregulation of the HPA axis has frequently reported in patients with psychiatric disorders, and is among the most robustly demonstrated neurobiological changes among psychiatric patients (D.A. Gutman and C.B. Nemeroff, Biological Psychiatry, 2002). The resulting elevated plasma cortisol concentrations leads to an enhanced binding of serotonin for the 5-HT2A receptor (E. A. Young, Arch Gen Psychiatry / Vol 60, Jan 2003).

Results suggest that cortical D2 dopamine receptors are a common target of traditional and atypical antipsychotics for therapeutic action. Higher in vivo binding to the D2 receptors in the cortex than in the basal ganglia is suggested as an indicator of favourable profile for a putative antipsychotic compound (X. Xiberas and J.L. Martinot; The British Journal of Psychiatry (2001) 179: 503-508).

Data demonstrate that dopamine D4 receptors play an important role in the induction of behavioral sensitization to amphetamine and accompanying adaptations in pre- and postsynaptic neural systems associated with the mesolimbocortical dopamine projections (D. L. Feldpausch et al; The journal of pharmacology and experimental therapeutics Vol. 286, Issue 1, 497-508, July 1998). Further, results show that dopamine D4 receptor antagonism in the brain does not result in the same neurochemical consequences (increased dopamine metabolism or hyperprolactinemia) observed with typical neuroleptics (Smita Patel et al; The journal of pharmacology and experimental therapeutics Vol. 283, Issue 2, 636-647, 1997).

However, the selective D4 dopamine receptor antagonist L-745,870 was ineffective as an antipsychotic for the treatment of neuroleptic responsive inpatients with acute schizophrenia (Kramer MS et al; Arch Gen Psychiatry 1997 Dec;54(12):1080.)

There is thus a growing need for a more efficient therapy and more efficient, selective and efficacious medicaments for treating mental disorders.

### Summary of the invention

The present invention invention relates to the use of compounds and pharmaceutical compositions having D4 and/or 5-HT2A antagonistic, partial agonistic or inverse agonistic activity for the treatment of the underlying dysregulation of the emotional functionality of mental disorders (i.e. affect instability - hypersensitivity - hyperaesthesia - dissociative phenomena - ...) and to methods entailing administering to a patient diagnosed as having a neuropsychiatric disorder a pharmaceutical composition containing (i) compounds having D4 antagonistic, partial agonistic or inverse agonistic activity and/or (ii) compounds having 5-HT2A antagonistic, partial agonistic or inverse agonistic, and/or (iii) any known medicinal compound and compositions of compounds. The combined D4 and 5-HT2A antagonistic, partial agonistic or inverse agonistic effects may reside within the same chemical or biological compound or in two different chemical or two different biological compounds or in a combination of a chemical & biological compound.

In a first embodiment, the invention relates to the use of a compound for the preparation of a medicament for treating a disease or disorder with an underlying dysregulation of the emotional functionality, characterised in that said compound has (i) a selective affinity for the Dopamine-4 (D4) receptor with a pKi value equal to or higher than 8 towards the D4 receptor and less than 8 towards other Dopamine receptors, and (ii) a selective affinity for the 5-HT2A receptor with a pKi value equal to or higher than 8 towards the 5-HT2A receptor and less than 8 towards other 5HT receptors and wherein said compound is administered to a patient in a dose ranging between 5 and 15 mg of the active ingredient. Preferably, said compound is PIPAMPERONE.

In a preferred embodiment, the invention relates to the use of a compound as defined above for preparing a medicament for treating a disease or disorder selected from the group comprising anxiety disorders, eating disorders, premenstrual syndrome, somatoform disorders, factitious disorders, dissociative disorders, sexual and gender identity disorders, sleep disorders, adjustment disorders, cognitive disorders, impulse control disorders, pervasive development, attention-deficit and disruptive behaviour disorders, substance-related disorders, personality disorders, psychological factors affecting medical conditions, malingering, antisocial behaviour, bereavement, occupational, identity, phase of life, academic problem, problems related to abuse or neglect.

According to a further embodiment the invention relates to the use of a first compound as defined above for the preparation of a medicament for treating a disease or disorder with an underlying dysregulation of the emotional functionality whereby a second compound is administered simultaneously with, separate from or sequential to said first compound to augment the therapeutic effect of said second compound on said disease, or to provide a faster onset of the therapeutic effect of said second compound on said disease. Preferably the disease or disorder to be treated is selected from the group comprising mood disorders, anxiety disorders, schizophrenia and other psychotic disorders, eating disorders, premenstrual syndrome, somatoform disorders, factitious disorders, dissociative disorders, sexual and gender identity disorders, sleep disorders, adjustment disorders, cognitive disorders, impulse control disorders, pervasive development, attention-deficit and disruptive behaviour disorders, substance-related disorders, personality disorders, psychological factors affecting medical conditions, malingering, antisocial behaviour, bereavement, occupational, identity, phase of life, academic problem, problems related to abuse or neglect.

In a preferred embodiment, the first compound is administered daily at least one day before administering said second compound. Preferably, said second compound is a selective serotonin re-uptake inhibitor, for instance chosen from the group comprising, but not limited to, CITALOPRAM, fluoxetine, venlafaxine, fluvoxamine, paroxetine, sertraline, milnacipran and duloxetine, or a pro-drug or an active metabolite thereof, or a pharmaceutically acceptable salt thereof. More preferably, said second compound is CITALOPRAM and is administered in a dose ranging between 10 and 40 mg of the active ingredient.

According to another preferred embodiment, said second compound is a nor-epinephrine re-uptake inhibitor, preferably chosen from the group comprising but not limited to tandamine, pirandamine, ciclazindol, fluparoxan, lortalamine, talsupram, talopram, prindamine, nomifensine, viloxazine, tomoxetine, duloxetine, venlafaxine, milnacipran and reboxetine, or a pro-drug or an active metabolite thereof, or a pharmaceutically acceptable salt thereof.

According to another preferred embodiment, said second compound is a neuroleptic agent, preferably chosen from the group comprising but not limited to chlorpromazine, haloperidol, perphenazine, thioridazine, mesoridazine, trifluoperazine, fluphenazine, clozapine, olanzapine, risperidone, ziprasidone, quetiapine, sertindole, aripiprazole, sonepiprazole, blonanserin, iloperidone, perospirone, raclopride, zotepine, DU-127090, ORG-5222, SM-13496, amisulpride, CP-361428, Lu 35-138, balaperidone, S-18327, WAY-135452, eplivanserin, E-5842, SR-31742, NE-100, osanetant, SR-141716, SR-48692, BSF-201640, BSF-190555, LAX-101a, sarizotan, CX-691 and SB-271046, or a pro-drug or an active metabolite thereof, or a pharmaceutically acceptable salt thereof.

The present invention also relates to the use of a pharmaceutical composition for the preparation of a medicament for treating a disease or disorder with an underlying dysregulation of the emotional functionality, for instance for treating a disease selected from the group comprising mood disorders, anxiety disorders, schizophrenia and other psychotic disorders, eating disorders, premenstrual syndrome, somatoform disorders, factitious disorders, dissociative disorders, sexual and gender identity disorders, sleep disorders, adjustment disorders, cognitive disorders, impulse control disorders, pervasive development, attention-deficit and disruptive behaviour disorders, substance-related disorders, personality disorders, psychological factors affecting medical conditions, malingering, antisocial behaviour, bereavement, occupational, identity, phase of life, academic problem, problems related to abuse or neglect; characterised in that said composition comprises a first compound having (i) a selective affinity for the D4 receptor with a pKi value equal to or higher than 8 towards the D4 receptor and less than 8 towards other Dopamine receptors, and a second compound having (ii) a selective affinity for the 5-HT2A receptor with a pKi value equal to or higher than 8 towards the 5-HT2A receptor and less than 8 towards other 5HT receptors.

In a preferred embodiment, said first compound of the composition is chosen from the group comprising PIPAMPERONE, FANANSERIN, L-745,870, PNU-101387G and U-101387 and said second compound of the composition is chosen from the group comprising PIPAMPERONE, FANANSERIN, ORG 5222, ZOTEPINE, OLANZEPINE, CLOZAPINE, S16924, S18327, AMPEROZIDE, SERTINDOLE, MDL 100.907, TIOSPIRONE, FLUSPIRILENE, OCAPERIDONE, RISPERIDONE and ZIPRASIDONE or a pro-drug or an active metabolite thereof, or a pharmaceutically acceptable salt thereof.

In a more preferred embodiment of the invention, said composition is administered to a patient in a dose ranging between 0.5 µg and 300 mg for each of the active ingredients.

According to the invention, for the following compounds, the following doses (daily doses) are preferred: PIPAMPERONE: 5 to 15 mg; ORG 5222: 1-10 mg; OLANZEPINE: 1-10 mg; CLOZAPINE: 1-200 mg; SERTINDOLE: 0.5-4 mg; OCAPERIDONE: 0.5-2 MICROGRAM; RISPERIDONE: 0.5-2 MG and ZIPRASIDONE: 1-20 MG.

According to yet another embodiment the invention relates to the use of a composition as defined above for the preparation of a medicament for treating a disease or disorder with an underlying dysregulation of the emotional functionality, for instance for treating a disease selected from the group comprising mood disorders, anxiety disorders, schizophrenia and other psychotic disorders, eating disorders, premenstrual syndrome, somatoform disorders, factitious disorders, dissociative disorders, sexual and gender identity disorders, sleep disorders, adjustment disorders, cognitive disorders, impulse control disorders, pervasive development, attention-deficit and disruptive behaviour disorders, substance-related disorders, personality disorders, psychological factors affecting medical conditions, malingering, antisocial behaviour, bereavement, occupational, identity, phase of life, academic problem, problems related to abuse or neglect; whereby said composition is administered simultaneously with, separate from or sequential to a third compound to augment the therapeutic effect of said third compound on said disease, or to provide a faster onset of the therapeutic effect of said third compound on said disease.

According to a preferred embodiment, said third compound is a selective serotonin re-uptake inhibitor. In a preferred embodiment, the first and second compound are administered daily at least one day before administering said third compound. Preferably, said third compound is a selective serotonin re-uptake inhibitor chosen from the group comprising but not limited to CITALOPRAM, fluoxetine, venlafaxine, fluvoxamine, paroxetine, sertraline, milnacipran and duloxetine, or a pro-drug or an active metabolite thereof, or a pharmaceutically acceptable salt thereof. More preferably, said third compound is CITALOPRAM and is administered in a dose ranging between 10 and 40 mg of the active ingredient.

According to another preferred embodiment, said third compound is a nor-epinephrine re-uptake inhibitor, preferably chosen from the group comprising but not limited to tandamine, pirandamine, ciclazindol, fluparoxan, lortalamine, talsupram, talopram, prindamine, nomifensine, viloxazine, tomoxetine, duloxetine, venlafaxine, milnacipran and reboxetine, or a pro-drug or an active metabolite thereof, or a pharmaceutically acceptable salt thereof.

According to another preferred embodiment, said third compound is a neuroleptic agent, preferably chosen from the group comprising but not limited to chlorpromazine, haloperidol, perphenazine, thioridazine, mesoridazine, trifluoperazine, fluphenazine, clozapine, olanzapine, risperidone, ziprasidone, quetiapine, sertindole, aripiprazole, sonepiprazole, blonanserin, iloperidone, perospirone, raclopride, zotepine, DU-127090, ORG-5222, SM-13496, amisulpride, CP-361428, Lu 35-138, balaperidone, S-18327, WAY-135452, eplivanserin, E-5842, SR-31742, NE-100, osanetant, SR-141716, SR-48692, BSF-201640, BSF-190555, LAX-101a, sarizotan, CX-691 and SB-271046, or a pro-drug or an active metabolite thereof, or a pharmaceutically acceptable salt thereof.

In a further embodiment, the invention also relates to the use of a compound having (i) a selective affinity for the Dopamine-4 (D4) receptor with a pKi value equal to or higher than 8 towards the D4 receptor and less than 8 towards other Dopamine receptors, and having (ii) a selective affinity for the 5-HT2A receptor with a pKi value equal to or higher than 8 towards the 5-HT2A receptor and less than 8 towards other 5HT receptors, or a composition comprising a first compound having a selective affinity for the Dopamine-4 (D4) receptor with a pKi value equal to or higher than 8 towards the D4 receptor and less than 8 towards other Dopamine receptors, and a second compound having a selective affinity for the 5-HT2A receptor with a pKi value equal to or higher than 8 towards the 5-HT2A receptor and less than 8 towards other 5HT receptors, for the preparation of a medicament for treating a musculoskeletal disease or disorder, characterised in that said compound or said composition is administered simultaneously with, separate from or sequential to a COX-2 inhibitor to augment the therapeutic effect of said COX-2 inhibitor, or to provide a faster onset of the therapeutic effect of said COX-2 inhibitor.

According to a preferred embodiment, the musculoskeletal disease or disorder to be treated is selected from the group comprising, but not limited to, rheumatoid arthritis, osteoarthritis or ankylosing spondylitis.

In a further preferred embodiment, the COX-2 inhibitor is chosen from the group comprising, but not limited to, celecoxib, rofecoxib, meloxicam, piroxicam, deracoxib, parecoxib, valdecoxib, etoricoxib, a chromene derivative, a chroman derivative, N-(2-cyclohexyloxynitrophenyl)-methane sulfonamide, COX189, ABT963 and JTE-522, or a pro-drug or an active metabolite thereof, or a pharmaceutically acceptable salt thereof.

The invention also relates to a method for preparing a compound having a selective D4 and 5-HT2A antagonist, reverse agonist or partial agonist activity comprising the following steps: (a) measuring the selective affinity of a test compound to the D4 receptor and selecting a compound that has a pKi value equal to or greater than 8 towards the D4 receptor in respect to all the other D receptors, and measuring the selective efficacy of the selected compound to the D4 receptor and selecting a compound which is a selective antagonist, inverse agonist or partial agonist of the D4 receptor; (b) measuring the selective affinity of a test compound to the 5-HT2A receptor and selecting a compound that has a pKi value equal to or greater than 8 towards the 5-HT2A receptor in respect to all the other 5HT receptors, and measuring the selective efficacy of the selected compound to the 5-HT2A receptor and selecting a compound which is a selective antagonist, inverse agonist or partial agonist of the 5-HT2A receptor; (c) identifying a compound which is selected in (a) and (b), (d) preparing the compound identified in (c).

The invention further also relates to a compound prepared by the described method.

### Detailed description of the invention

The present inventors surprisingly found that compounds which have a high selective affinity towards the 5-HT2A receptor and which, at the same time have a high selective affinity towards the Dopamine-4 (D4) receptor show an improved effect in treating underlying dysregulation of the emotional functionality of mental disorders.

The compounds according to the invention may be chemical or biological in nature, or may be chemically synthesised. Preferably, the compounds of the invention are provided as a pharmaceutically acceptable salt.

One example of such a compound which has both a selective affinity for the 5-HT2A receptor with a pKi value equal to or higher than 8 towards the 5-HT2A receptor and less than 8 towards other 5HT receptors, and a selective affinity for the D4 receptor with a pKi value equal to or higher than 8 towards the D4 receptor and less than 8 towards other Dopamine receptors is PIPAMPERONE. PIMPAPERONE is the conventional name given for the compound of the formula 1'-[3-(p-Fluorobenzoyl)propyl]-[1,4'-bipiperidine]-4'-carboxamide. PIPAMPERONE is also the active ingredient of Dipiperon (Janssen, Cilag B.V).

Further, the present inventors surprisingly found that the dosage of active ingredient for PIPAMPERONE in treatment could be very low compared to conventionally used dosages. Preferred dosages which, according to the invention, have been shown to be effective for treating these mental disorders, range between 5 and 15 mg per day or between 5 and 10 mg per day. More preferably, dosages of 5, 6, 7, 8, 9, 10, 11, 12, 13, 14 or 15 mg per day are used in treatment of the diseases of the invention. In conventional PIPAMPERONE treatment, the active ingredient is available in tablets of 40 mg per tablet or in solutions of 2 mg per drop. Conventional usage of high doses ranging from 40 to 360 mg is prescribed. For instance for children up to the age of 14, a doses corresponding with 2 to 6 mg per kg body weight is conventionally prescribed. The high selective affinity of PIPAMPERONE towards the 5-HT2A receptor and the D4 receptor is reflected in the low dosage which is needed for the treatment of the mental diseases listed below and also contributes to the efficacy of the treatment.

The mental disorders which can be treated using PIPAMPERONE in a mono therapy at such low doses are for instance anxiety disorders, eating disorders, premenstrual syndrome, somatoform disorders, factitious disorders, dissociative disorders, sexual and gender identity disorders, sleep disorders, adjustment disorders, cognitive disorders, impulse control disorders, pervasive development, attention-deficit and disruptive behaviour disorders, substance-related disorders, personality disorders, psychological factors affecting medical conditions, malingering, antisocial behaviour, bereavement, occupational, identity, phase of life, academic problem, problems related to abuse or neglect.

Mental disorders such as depression are commonly treated with serotonin re-uptake inhibitors. Unfortunately, however, these compounds can give rise to side effects in use. Moreover, a substantial problem in most treatment of mental disorders is the non-response to selective serotonin re-uptake inhibitors (SSRIs). Also the onset of the therapeutic effect can be delayed undesirable.

A problem to be solved by the present invention is thus the provision of a more efficient therapy and efficient, highly selective and efficacious medicaments for treating mental disorders.

The inventors found that the non-response to selective serotonin re-uptake inhibitors (SSRIs) in depression may be declared by (partial) inhibition of the 5-HT1A stimulation via 5-HT2A stimulation. Des-inhibition thereof via 5-HT2A antagonism seems to be an answer to this problem.

The present inventors found that a simultaneous or foregoing treatment with a compound having a high selective 5-HT2A antagonist, inverse agonist or partial agonist activity, could lead to a greater response towards SSRIs. However, not all compounds exhibiting 5-HT2A antagonism are useful: competition between 5-HT2A stimulation via serotonin and 5-HT2A antagonism via the compound could be responsible for the lack of more efficacy of compounds which have both a selective serotonin re-uptake inhibitory and 5-HT2A antagonist profile, such as trazodone and nefazodone.

The present inventors further surprisingly found that a simultaneous or foregoing treatment with a compound having a high selective D4 antagonist, inverse agonist or partial agonist activity in combination with a compound having a high selective 5-HT2A antagonist, inverse agonist or partial agonist activity could lead to a greater response towards SSRIs.

The present inventors found that a compound which binds to the 5-HT2A receptor with a pKi of at least 8 but for which the binding affinity, ie pKi, towards other 5HT receptors is less than 8 in combination with a compound which has a high selective affinity for the D4 receptor, i.e. which bind to the D4 receptor with a pKi of at least 8 but for which the binding affinity, ie pKi, towards other dopamine receptors is less than 8 also show such an improved effect in treatment. These effects, ie D4 antagonism, inverse agonism or partial agonism and 5-HT2A antagonism, inverse agonism or partial agonism, preferably reside in the same compound. In other embodiments of the invention, these effects reside in separate compounds.

'Other 5HT receptors' as used herein are for instance 5-HT1 receptors (i.e. 5-HT1A, 5-HT1B, 5-HT1D, 5-HT1E, 5-HT1F), 5-HT2B, 5-HT2C, 5-HT6 (rat) and 5-HT7 (rat).

5-HT2A responsive compounds according to the invention are, for instance PIPAMPERONE, FANANSERIN, L-745,870, PNU-101387G and U-101387. All these compounds are known in the art and are to be used in doses according to the supplier's or physician's prescription.

By the expression 'selective affinity for the 5-HT2A receptor' is meant that the receptor has a higher affinity for the 5-HT2A receptor than for other 5-HT receptors.

Preferably, the compounds of the invention which have a selective affinity for the 5-HT2A receptor, are compounds which have a pKi value equal to or higher than 8 towards the 5-HT2A receptor and less than 8 towards other 5HT receptors, as can be measured, for instance by methods known in the art. For instance, the "NIMH Psychoactive Drug Screening Program (PDSP)" Kᵢ database (http://kidb.cwru.edu/nimh/5htp.php), is a unique resource in the public domain which provides information on the abilities of drugs to interact with an expanding number of molecular targets. The PDSP Kᵢ database serves as a data warehouse for published and internally-derived pKi, or affinity, values for a large number of drugs and drug candidates at an expanding number of G-protein coupled receptors, ion channels, transporters and enzymes. The PDSP internet site also provides for commonly used protocols and assays for measuring pKi values of 5HT receptors.

The expression 'selective affinity for the D4 receptor' means that the receptor has a higher affinity for the Dopamine D4 receptor than for other Dopamine receptors.

D4 responsive compounds according to the invention are, for instance, PIPAMPERONE, FANANSERIN, ORG 5222, ZOTEPINE, OLANZEPINE, CLOZAPINE, S16924, S18327, AMPEROZIDE, SERTINDOLE, MDL 100.907, TIOSPIRONE, FLUSPIRILENE, OCAPERIDONE, RISPERIDONE and ZIPRASIDONE. All these compounds are known in the art and are to be used in doses according to the supplier's or physician's prescription.

'Other Dopamine receptors' are, for instance, D1, D2 and D3.

pKi values of test compounds for Dopamine receptors can be measured using commonly known assays.

Compounds which have a selective affinity for the D4 receptor preferably have a pKi value equal to or higher than 8 towards the D4 receptor and less than 8 towards other Dopamine receptors.

A preferred example of a compound which has both a selective affinity for the 5-HT2A receptor with a pKi value equal to or higher than 8 towards the 5-HT2A receptor and less than 8 towards other 5HT receptors, and a selective affinity for the D4 receptor with a pKi value equal to or higher than 8 towards the D4 receptor and less than 8 towards other Dopamine receptors and which is therefore useful in a combination therapy is PIPAMPERONE.

Table 1 illustrates the selective affinity of for instance PIPAMPERONE for the 5-HT2A and for the D4 receptor. In addition, Table 1 also illustrates the low or absence of affinity of PIPAMPERONE for other receptors such as the adrenergic receptors Alpha 1A, Alpha 2A, Alpha 2B, Alpha 2C, Beta1, Beta2, and the histamine receptor H1. As such, treating patients with PIPAMPERONE will provide for less side effects which otherwise result from simultaneous stimulation of other receptors. Therefore, and according to preferred embodiments, useful compounds according to the invention not only have a selective 5-HT2A and/or D4 affinity but also a low affinity for other receptors such as the adrenergic and histamine receptors.

The low dosage which can be used in PIPAMPERONE treatment, as already described earlier, contributes to the high selective affinity of the compound towards the 5-HT2A receptor and the D4 receptor and therefore also to the efficacy of the treatment.

The mental disorders which can be treated using compounds having a high selective affinity for the 5-HT2A and D4 receptor, for instance PIPAMPERONE, in a combination therapy with an SSRI are for instance mood disorders, anxiety disorders, schizophrenia and other psychotic disorders, eating disorders, premenstrual syndrome, somatoform disorders, factitious disorders, dissociative disorders, sexual and gender identity disorders, sleep disorders, adjustment disorders, cognitive disorders, impulse control disorders, pervasive development, attention-deficit and disruptive behaviour disorders, substance-related disorders, personality disorders, psychological factors affecting medical conditions, malingering, antisocial behaviour, bereavement, occupational, identity, phase of life, academic problem, problems related to abuse or neglect.

These diseases and their diagnosis are very clearly defined in the "Diagnostic and Statistical Manual of Mental Disorders" published by the American Psychiatric Association. This manual sets forth diagnostic criteria, descriptions and other information to guide the classification and diagnosis of mental disorders and is commonly used in the field of neuropsychiatry. It is for instance available on the internet under: http://www.behavenet.com/capsules/disorders/dsm4tr.htm.

According to a preferred embodiment, the invention thus relates to the use of a compound having a high selective affinity for the 5-HT2A and D4 receptor in combination with a selective serotonin re-uptake inhibiter, for instance chosen from the group comprising CITALOPRAM, fluoxetine, venlafaxine, fluvoxamine, paroxetine, sertraline, milnacipran and duloxetine or a prodrug or an active metabolite thereof, or a pharmaceutically acceptable salt thereof.

The terms "treatment", "treating", and the like, as used herein include amelioration or elimination of a developed mental disease or condition once it has been established or alleviation of the characteristic symptoms of such disease or condition. As used herein these terms also encompass, depending on the condition of the patient, preventing the onset of a disease or condition or of symptoms associated with a disease or condition, including reducing the severity of a disease or condition or symptoms associated therewith prior to affliction with said disease or condition. Such prevention or reduction prior to affliction refers to administration of the compound or composition of the invention to a patient that is not at the time of administration afflicted with the disease or condition. "Preventing" also encompasses preventing the recurrence or relapse-prevention of a disease or condition or of symptoms associated therewith, for instance after a period of improvement. It should be clear that mental conditions may be responsible for physical complaints. In this respect, the term "treating" also includes prevention of a physical disease or condition or amelioration or elimination of the developed physical disease or condition once it has been established or alleviation of the characteristic symptoms of such conditions.

As used herein, the term "medicament" also encompasses the terms "drug", "therapeutic", "potion" or other terms which are used in the field of medicine to indicate a preparation with therapeutic or prophylactic effect.

The present inventors not only found that the selective 5-HT2A and D4 antagonists, inverse agonists or partial agonists have an effect in augmenting the therapeutic effect or in providing a faster onset of the therapeutic effect of selective serotonin re-uptake inhibitors, but also that this effect is seen in therapy with other pharmaceutical compounds. A few examples of other pharmaceutical compounds whose effects are augmented or where the onset of the effect is fastened upon simultaneous or fore-going treatment with a selective 5-HT2A and D4 antagonist, inverse agonist or partial agonist, are nor-epinephrine re-uptake inhibitors, neuroleptic agents or compounds used for treating or alleviating musculoskeletal diseases or disorders. It should be clear, given the general applicable character of the invention, that this list of other pharmaceutical compounds is very brief and that the invention should not be restricted to the ones exemplified herein.

According to the invention it thus has been found that the compounds having a selective 5-HT2A and D4 antagonist, inverse agonist or partial agonist activity as described above are useful for augmenting the therapeutic effect of a second compound on a disease.

According to another embodiment of the invention it has also been found that the compounds having a selective 5-HT2A and D4 antagonist, inverse agonist or partial agonist activity as described above are useful for providing a faster onset of the therapeutic effect of a second compound on a disease.

In one embodiment, the compound having a selective 5-HT2A and D4 antagonist, inverse agonist or partial agonist activity is used in a combination therapy with the second compound are to treat the same disease or disorder, for instance a disease selected from the group comprising mood disorders, anxiety disorders, schizophrenia and other psychotic disorders, eating disorders, premenstrual syndrome, somatoform disorders, factitious disorders, dissociative disorders, sexual and gender identity disorders, sleep disorders, adjustment disorders, cognitive disorders, impulse control disorders, pervasive development, attention-deficit and disruptive behaviour disorders, substance-related disorders, personality disorders, psychological factors affecting medical conditions, malingering, antisocial behaviour, bereavement, occupational, identity, phase of life, academic problem, problems related to abuse or neglect.

Preferably, said second compound is a nor-epinephrine re-uptake inhibitor. Preferred nor-epinephrine re-uptake inhibitors to be administered in combination with the selective 5-HT2A and D4 antagonist, inverse agonist or partial agonist of the invention are chosen from the group comprising tandamine, pirandamine, ciclazindol, fluparoxan, lortalamine, talsupram, talopram, prindamine, nomifensine, viloxazine, tomoxetine, duloxetine, venlafaxine, milnacipran and reboxetine, pharmaceutically acceptable salts, pro-drugs and mixtures thereof. In other preferred embodiments, said second compound is a neuroleptic agent. Preferred neuroleptic agents to be administered in combination with the selective 5-HT2A and D4 antagonist, inverse agonist or partial agonist of the invention are chosen from the group comprising chlorpromazine, haloperidol, perphenazine, thioridazine, mesoridazine, trifluoperazine, fluphenazine, clozapine, olanzapine, risperidone, ziprasidone, quetiapine, sertindole, aripiprazole, sonepiprazole, blonanserin, iloperidone, perospirone, raclopride, zotepine, DU-127090, ORG-5222, SM-13496, amisulpride, CP-361428, Lu 35-138, balaperidone, S-18327, WAY-135452, eplivanserin, E-5842, SR-31742, NE-100, osanetant, SR-141716, SR-48692, BSF-201640, BSF-190555, LAX-101a, sarizotan, CX-691 and SB-271046, pharmaceutically acceptable salts, pro-drugs or active metabolites thereof, or mixtures thereof.

In alternative embodiments, the second compound is used to treat another disease. In a preferred embodiment, said second compound is a COX-2 inhibitor and is used for treating musculoskeletal diseases or for the management of acute pain or for primary treatment of dysmenorrhea, and the first compound augments the therapeutic effect or provides for a faster onset of the therapeutic effect of said second compound on said other disease.

Preferred COX-2 inhibitors to be administered in combination with the selective 5-HT2A and D4 antagonist, inverse agonist or partial agonist of the invention are chosen from the group comprising celecoxib, rofecoxib, meloxicam, piroxicam, deracoxib, parecoxib, valdecoxib, etoricoxib, a chromene derivative, a chroman derivative, N-(2-cyclohexyloxynitrophenyl)-methane sulfonamide, COX189, ABT963 or JTE-522, pharmaceutically acceptable salts, pro-drugs or active metabolites thereof, or mixtures thereof.

From the above it should be clear that the selective 5-HT2A and D4 antagonist, inverse agonist or partial agonist is also named 'the first compound' in the embodiments of the invention.

According to the invention, when the 5-HT2A and D4 antagonist, inverse agonist or partial agonist activity reside in separate compounds, the term "composition" will be used. Compositions of the invention comprise a first compound having (i) a selective affinity for the D4 receptor with a pKi value equal to or higher than 8 towards the D4 receptor and less than 8 towards other Dopamine receptors, and a second compound having (ii) a selective affinity for the 5-HT2A receptor with a pKi value equal to or higher than 8 towards the 5-HT2A receptor and less than 8 towards other 5HT receptors.

The expression "the 5-HT2A and D4 antagonist, inverse agonist or partial agonist" is used herein to indicate a single compound having both activities or to indicate the composition comprising the activity in separate compounds.

It should be clear that when, in the present invention, a composition of separate compounds is used instead of a single compound, they may be used in combination with another, i.e. a third, compound to augment the therapeutic effect of the other, i.e. the third, compound on the same or another disease.

When the 5-HT2A and D4 antagonist, inverse agonist or partial agonist and the second compound or third compound, are administered simultaneously, the compounds or active ingredients may be present in a single pharmaceutical composition or formulation. Alternatively the compounds or active ingredients are administered in separate pharmaceutical compositions or formulations for simultaneous or separate use.

When the 5-HT2A and D4 antagonist, inverse agonist or partial agonist of the invention is administered prior to the second or third compound, as defined, the 5-HT2A and D4 antagonist, inverse agonist or partial agonist is administered at least during 1 day prior to said second or third compound. Preferably the 5-HT2A and D4 antagonist, inverse agonist or partial agonist is administered for at least 1, 2 3, 4, 5, 6, 7, 8, 9 or 10 days, prior to the administration of the second or third compound. Preferably the 5-HT2A and D4 antagonist, inverse agonist or partial agonist is administered for at least 2, 3, 4, 5 weeks prior to the administration of the second or third compound, or for at least 1, 2, 3, 4, 5, 6, 7, 8, 9, 10, 11 or 12 months prior to the administration of the second or third compound.

According to a preferred embodiment of the invention, the above described compounds having a 5-HT2A and D4 antagonist, inverse agonist or partial agonist activity are useful for augmenting the therapeutic effect of CITALOPRAM or for providing a faster onset of the therapeutic effect of CITALOPRAM.

CITALOPRAM or citalopram hydrobromide is a selective serotonin (5-hydroxytryptamine / 5-HT) re-uptake inhibitor (SSRI) and is the conventional name given for the compound of the formula (RS)-1-[3-(dimethylamino)propyl]-1-(*p*-flurophenyl)-5-phthalancarbonitrile, hydro-bromide.

According to one embodiment, a daily doses of active ingredient of SSRI, preferably CITALOPRAM, ranges between 10 and 40 mg per day. Preferably, daily doses of active ingredient ranging between 20 and 30 mg per day are administered. More preferably, a daily dose of 10, 15, 20, 25, 30, 35 or 40 mg per day is administered.

Other preferred second or third compounds according to the invention are chosen from the group comprising selective serotonin re-uptake inhibitors, for instance CITALOPRAM, fluoxetine, venlafaxine, fluvoxamine, paroxetine, sertraline, milnacipran and duloxetine; COX-2 inhibitors, for instance celecoxib, rofecoxib, meloxicam, piroxicam, deracoxib, parecoxib, valdecoxib, etoricoxib, a chromene derivative, a chroman derivative, N-(2-cyclohexyloxynitrophenyl)methane sulfonamide, COX189, ABT963 or JTE-522;nor-epinephrine re-uptake inhibitors, for instance tandamine, pirandamine, ciclazindol, fluparoxan, lortalamine, talsupram, talopram, prindamine, nomifensine, viloxazine, tomoxetine, duloxetine, venlafaxine, milnacipran and reboxetine; and neuroleptic agents, for instance chlorpromazine, haloperidol, perphenazine, thioridazine, mesoridazine, trifluoperazine, fluphenazine, clozapine, olanzapine, risperidone, ziprasidone, quetiapine, sertindole, aripiprazole, sonepiprazole, blonanserin, iloperidone, perospirone, raclopride, zotepine, DU-127090, ORG-5222, SM-13496, amisulpride, CP-361428, Lu 35-138, balaperidone, S-18327, WAY-135452, eplivanserin, E-5842, SR-31742, NE-100, osanetant, SR-141716, SR-48692, BSF-201640, BSF-190555, LAX-101a, sarizotan, CX-691 and SB-271046. All these compounds are known in the art and are to be used in doses according to the supplier's or physician's prescription.

Also encompassed by the invention are pro-drugs to these second or third compounds or active metabolites of these compounds. For instance, for risperidone it is known that, among other products, biotransformation in the liver produces 9-hydroxyrisperidone, which is of the same pharmacological activity and intensity as parent risperidone. Therefore, also 9-hydroxyrisperidone, naturally produced or chemically synthesized may be used in the methods and uses according to the invention.

The term "active metabolite" as used herein relates to a therapeutically active compound produced by the metabolism of a parent drug. Drugs administered to treat diseases are usually transformed (metabolized) within the body into a variety of related chemical forms (metabolites), some of which may have therapeutic activity (an active metabolite).

The present invention also encompasses the use of these second or third compounds, administered in the form of a pharmaceutically acceptable salt in admixture with a suitable pharmaceutically acceptable excipient.

To prepare the pharmaceutical compositions, comprising the compounds or the combination of the first and second compound described herein, an effective amount of the active ingredients, in acid or base addition salt form or base form, is combined in admixture with a pharmaceutically acceptable carrier, which can take a wide variety of forms depending on the form of preparation desired for administration. These pharmaceutical compositions are desirably in unitary dosage form suitable, for administration orally, nasal, rectally, percutaneously or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included.

According to a further embodiment, the invention also relates to a method for preparing a compound having a selective D4 and 5-HT2A antagonist, reverse agonist or partial agonist. The invention also relates to the compounds prepared by the claimed method, with the proviso that said compound is not an already known compound, such as PIPAMPERONE.

It should be clear that the compounds and compositions described herein are useful for treating any patient in need thereof. As used herein the term "patient" is not restricted to humans but also to other mammals, for instance domestic animals which may also suffer from any form of a mental disease or disorder described herein.

The invention, now being generally described, will be more readily understood by reference to the following tables and examples, which are included merely for purposes of illustration of certain aspects and embodiments of the present invention and are not intended to limit the invention.

### Examples

### Example 1: Measuring pKi values of test compounds

In Table 1, the pKi values of test compounds are given for each of the dopamine receptors, 5HT receptors, adrenergic receptors and the histamine1 receptor. The affinity of test compounds for the respective receptors has been performed according to conventional procedures known in the art.

An indication "0" means that no affinity has been measured between the test compound and the receptor.

The columns displaying the pKi values for the D4 and the 5-HT2A receptor are filled with dark grey. pKi values between 8 and 9 and higher than 9 are represented by light grey boxes.

### Example 2: Foregoing pipamperon-citalopram treatment in mayor depressive disorder: a placebo and active controlled period finding clinical trial

Table 2 represents the set-up of a clinical trial comprising for treatment groups:
Group Plc - Active / Day 0 represents the group receiving 10 mg citalopram, twice a day, starting the first day (Day 0) of active treatment in the clinical trial. This administration regime is also indicated as the mono therapy.
Group Pip - Active / Day 0 represents the group receiving a combination of 4 mg pipamperon and 10 mg citalopram, twice a day, starting the first day (Day 0) of active treatment in the clinical trial. This administration regime is also indicated as the non-foregoing combo therapy.
Group Pip - Active / Day 4 represents the group receiving 4 mg pipamperon, twice a day, starting the first day (Day 0) of active treatment in the clinical trial, followed by a combination of 4 mg pipamperon and 10 mg citalopram, twice a day, starting the fifth (Day 4) day of active treatment in the clinical trial. This administration regime is also indicated as the foregoing therapy with combination therapy starting after 4 days of active treatment.
Group Pip - Active / Day 7 represents the group receiving 4 mg pipamperon, twice a day, starting the first day (Day 0) of active treatment in the clinical trial, followed by a combination of 4 mg pipamperon and 10 mg citalopram, twice a day, starting the eight (Day 7) day of active treatment in the clinical trial. This administration regime is also indicated as the foregoing therapy with combination therapy starting after 7 days of active treatment.

All subjects also undergo a placebo (PLC) run-in therapy, administered during a period of about 7 days before the active treatment starts.

During daily (D), weekly (W) or monthly (M) visits, several parameters are measured.

Under NECT is to be understood: Neuronal E-clinical Trial = Vesalius Expert development for this trial which includes the bottom-up measurement of:
- In- and exclusion-criteria
- Functional status evaluation
- Medical history
- (Pre-)treatment signs & symptoms
- DSM-IV rules for diagnosis & efficacy
- HDRS-28 (Hamilton Depression Rating Scale - 28 items)
- Medical resource utilisation
- Pre-trial & Concomittant medication
- Drug administration
- (Serious) Adverse events
- Admission to the acute and extension phase of treatment
- Right flow of the trial

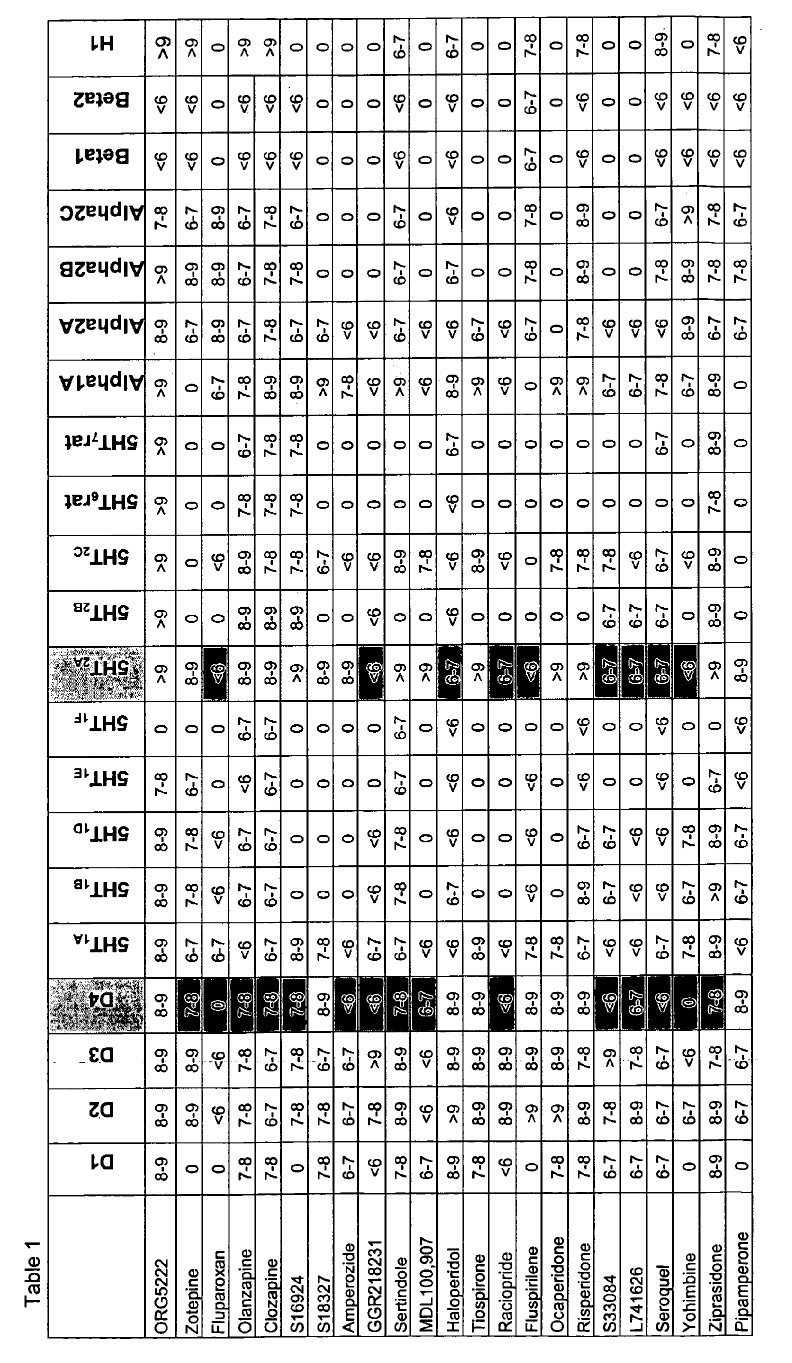

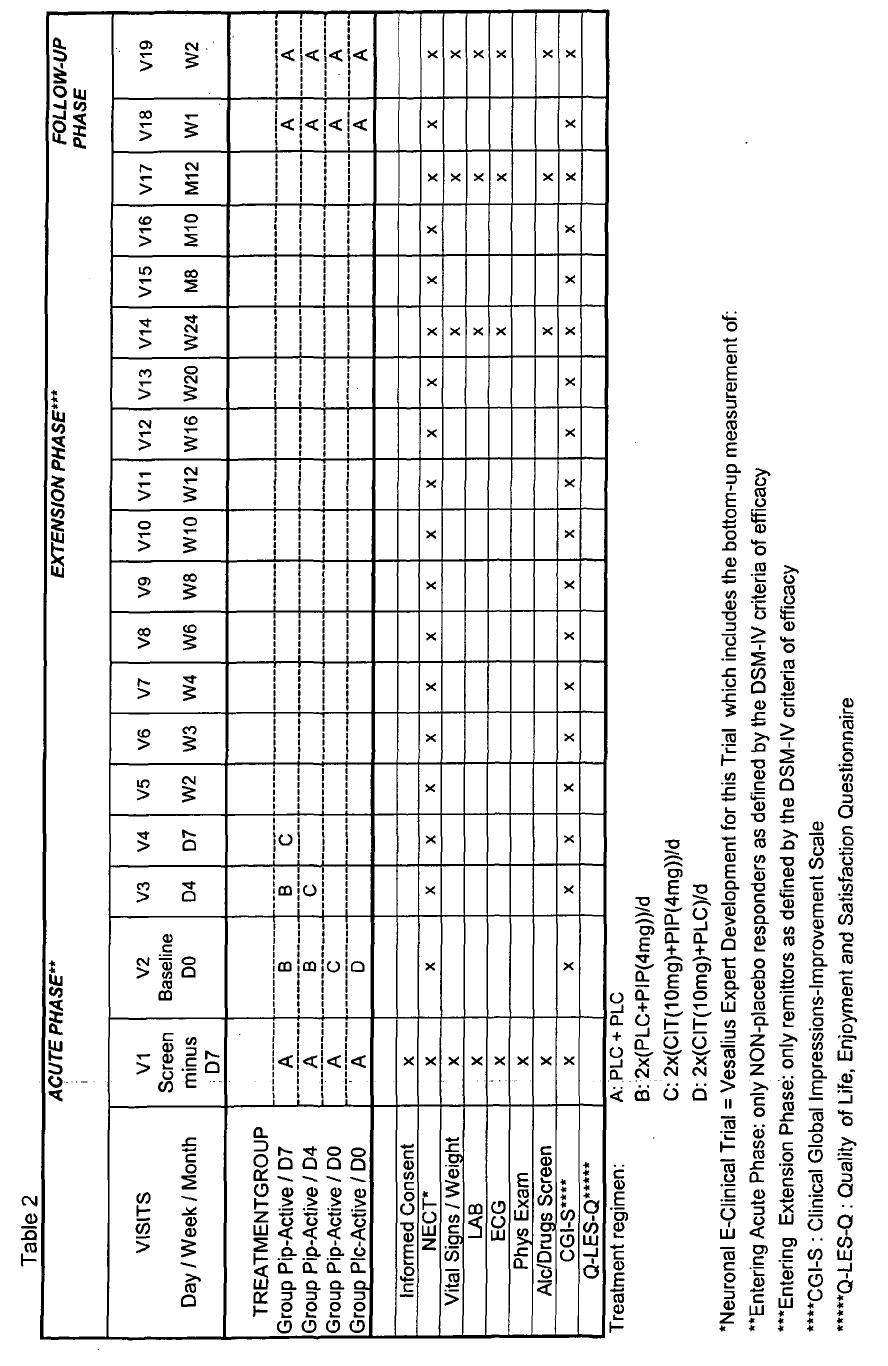

## Claims

1. Use of pipamperone for the preparation of a medicament for treating mood disorders, wherein said pipamperone is daily administered to a patient in a dose ranging between 5 and 15 mg of the active ingredient, and wherein a second compound is administered simultaneously with, separate from or sequential to said pipamperone.

2. The use according to claim 1, wherein said pipamperone is administered daily at least one day before administering said second compound.

3. The use according to claim 1 or 2, wherein said second compound is a selective serotonin re-uptake inhibitor.

4. The use according to claim 3, wherein said selective serotonin re-uptake inhibitor is chosen from the group comprising citalopram, fluoxetine, venlafaxine, fluvoxamine, paroxetine, sertraline, milnacipran and duloxetine or a pro-drug or a pharmaceutically acceptable salt thereof.

5. The use according to claim 4, wherein said serotonin re-uptake inhibitor is citalopram and is administered in a dose ranging between 10 and 40 mg of the active ingredient.

6. The use of pipamperone for the preparation of a medicament for treating a mood disorders according to claim 1 or 2, wherein said second compound is a nor-epinephrine re-uptake inhibitor.

7. The use according to claim 6, wherein said nor-epinephrine re-uptake inhibitor is chosen from the group comprising tandamine, fluparoxan, talsupram, talopram, viloxazine, tomoxetine, duloxetine, venlafaxine, milnacipran and reboxetine, or a pro-drug or a pharmaceutically acceptable salt thereof.

8. Use according to any of claims 4 to 6 wherein said second compound is a selective serotonin re-uptake inhibitor.

9. Use according to claim 8 wherein said selective serotonin re-uptake inhibitor is chosen from the group comprising CITALOPRAM, fluoxetine, venlafaxine, fluvoxamine, paroxetine, sertraline, milnacipran and duloxetine or a pro-drug or an active metabolite thereof, or a pharmaceutically acceptable salt thereof.

10. Use according to claim 9 wherein said serotonin re-uptake inhibitor is CITALOPRAM and is administered in a dose ranging between 10 and 40 mg of the active ingredient.

11. Use of a composition for the preparation of a medicament for treating a disease or disorder with an underlying dysregulation of the emotional functionality, **characterised in that** said composition comprises a first compound having (i) a selective affinity for the D4 receptor with a pKi value equal to or higher than 8 towards the D4 receptor and less than 8 towards other Dopamine receptors, and a second compound having (ii) a selective affinity for the 5-HT2A receptor with a pKi value equal to or higher than 8 towards the 5-HT2A receptor and less than 8 towards other 5HT receptors.

12. Use according to claim 11 wherein said disease or disorder is selected from the group comprising mood disorders, anxiety disorders, schizophrenia and other psychotic disorders, eating disorders, premenstrual syndrome, somatoform disorders, factitious disorders, dissociative disorders, sexual and gender identity disorders, sleep disorders, adjustment disorders, cognitive disorders, impulse control disorders, pervasive development, attention-deficit and disruptive behaviour disorders, substance-related disorders, personality disorders, psychological factors affecting medical conditions, malingering, antisocial behaviour, bereavement, occupational, identity, phase of life, academic problem, problems related to abuse or neglect.

13. Use according to claim 11 or 12 wherein said first compound is chosen from the group comprising PIPAMPERONE, FANANSERIN, L-745,870, PNU-101387G and U-101387 or a pro-drug or a pharmaceutically acceptable salt thereof and wherein said second compound is chosen from the group comprising PIPAMPERONE, FANANSERIN, ORG 5222, ZOTEPINE, OLANZEPINE, CLOZAPINE, S16924, S18327, AMPEROZIDE, SERTINDOLE, MDL 100.907, TIOSPIRONE, FLUSPIRILENE, OCAPERIDONE, RISPERIDONE and ZIPRASIDONE or a pro-drug or an active metabolite thereof, or a pharmaceutically acceptable salt thereof.

14. Use according to any of claims 11 to 13 wherein said composition is administered to a patient in a dose ranging between 0,5 µg and 300 mg for each of the active ingredients.

15. Use according to any of claims 11 to 14 wherein said composition is administered simultaneously with, separate from or sequential to a third compound to augment the therapeutic effect of said third compound.

16. Use according to any of claims 12 to 15 wherein said composition is administered simultaneously with, separate from or sequential to a third compound to provide a faster onset of the therapeutic effect of said third compound.

17. Use according to claim 15 or 16 wherein said third compound is a selective serotonin re-uptake inhibitor.

18. Use according to claim 17 wherein said selective serotonin re-uptake inhibitor is chosen from the group comprising CITALOPRAM, fluoxetine, venlafaxine, fluvoxamine, paroxetine, sertraline, milnacipran and duloxetine, or a pro-drug or an active metabolite thereof, or a pharmaceutically acceptable salt thereof.

19. Use according to claim 18 wherein said selective serotonin re-uptake inhibitor is CITALOPRAM and is administered in a dose ranging between 10 and 40 mg of the active ingredient.

20. Use of a compound as defined in claim 1 or of a composition as defined in claim 11 for the preparation of a medicament for treating a disease or disorder as defined in claim 12 with an underlying dysregulation of the emotional functionality, **characterised in that** said compound or composition is administered simultaneously with, separate from or sequential to a nor-epinephrine re-uptake inhibitor to augment the therapeutic effect of said nor-epinephrine re-uptake inhibitor.

21. Use of a compound as defined in claim 1 or of a composition as defined in claim 11 for the preparation of a medicament for treating a disease or disorder as defined in claim 12 with an underlying dysregulation of the emotional functionality, **characterised in that** said compound or composition is administered simultaneously with, separate from or sequential to a nor-epinephrine re-uptake inhibitor to provide a faster onset of the therapeutic effect of said nor-epinephrine re-uptake inhibitor.

22. Use according to claim 20 or 21 wherein said nor-epinephrine re-uptake inhibitor is chosen from the group comprising tandamine, pirandamine, ciclazindol, fluparoxan, lortalamine, talsupram, talopram, prindamine, nomifensine, viloxazine, tomoxetine, duloxetine, venlafaxine, milnacipran and reboxetine, or a pro-drug or an active metabolite thereof, or a pharmaceutically acceptable salt thereof.

23. Use of a compound as defined in claim 1 or of a composition as defined in claim 11 for the preparation of a medicament for treating a disease or disorder as defined in claim 12 with an underlying dysregulation of the emotional functionality, **characterised in that** said compound or composition is administered simultaneously with, separate from or sequential to a neuroleptic agent to augment the therapeutic effect of said neuroleptic agent.

24. Use of a compound as defined in claim 1 or of a composition as defined in claim 11 for the preparation of a medicament for treating a disease or disorder as defined in claim 12 with an underlying dysregulation of the emotional functionality, **characterised in that** said compound or composition is administered simultaneously with, separate from or sequential to a neuroleptic agent to provide a faster onset of the therapeutic effect of said neuroleptic agent.

25. Use according to claim 23 or 24 wherein said neuroleptic agent is chosen from the group comprising chlorpromazine, haloperidol, perphenazine, thioridazine, mesoridazine, trifluoperazine, fluphenazine, clozapine, olanzapine, risperidone, ziprasidone, quetiapine, sertindole, aripiprazole, sonepiprazole, blonanserin, iloperidone, perospirone, raclopride, zotepine, DU-127090, ORG-5222, SM-13496, amisulpride, CP-361428, Lu 35-138, balaperidone, S-18327, WAY-135452, eplivanserin, E-5842, SR-31742, NE-100, osanetant, SR-141716, SR-48692, BSF-201640, BSF-190555, LAX-101a, sarizotan, CX-691 and SB-271046, or a pro-drug or an active metabolite thereof, or a pharmaceutically acceptable salt thereof.

26. Use of a compound as defined in claim 1 or of a composition as defined in claim 11 for the preparation of a medicament for treating a musculoskeletal disease or disorder, **characterised in that** said compound or composition is administered simultaneously with, separate from or sequential to a COX-2 inhibitor to augment the therapeutic effect of said COX-2 inhibitor.

27. Use of a compound as defined in claim 1 or of a composition as defined in claim 11 for the preparation of a medicament for treating a musculoskeletal disease or disorder, **characterised in that** said compound or composition is administered simultaneously with, separate from or sequential to a COX-2 inhibitor to provide a faster onset of the therapeutic effect of said COX-2 inhibitor.

28. Use according to claim 26 or 27 wherein said disease or disorder is selected from the group comprising rheumatoid arthritis, osteoarthritis or ankylosing spondylitis.

29. Use according to any of claims 26 to 28 wherein said COX-2 inhibitor is chosen from the group comprising celecoxib, rofecoxib, meloxicam, piroxicam, deracoxib, parecoxib, valdecoxib, etoricoxib, a chromene derivative, a chroman derivative, N-(2-cyclohexyloxynitrophenyl)methane sulfonamide, COX189, ABT963 and JTE-522, or a pro-drug or an active metabolite thereof, or a pharmaceutically acceptable salt thereof.

30. A method for preparing a compound having a selective D4 and 5-HT2A antagonist, reverse agonist or partial agonist activity comprising the following steps: (a) measuring the selective affinity of a test compound to the D4 receptor and selecting a compound that has a pKi value equal to or greater than 8 towards the D4 receptor in respect to all the other D receptors, and measuring the selective efficacy of the selected compound to the D4 receptor and selecting a compounds which is a selective antagonist, inverse agonist or partial agonist of the D4 receptor; (b) measuring the selective affinity of a test compound to the 5-HT2A receptor and selecting a compound that has a pKi value equal to or greater than 8 towards the 5-HT2A receptor in respect to all the other 5HT receptors, and measuring the selective efficacy of the selected compound to the 5-HT2A receptor and selecting a compounds which is a selective antagonist, inverse agonist or partial agonist of the 5-HT2A receptor; (c) identifying a compound which is selected in (a) and (b), (d) preparing the compound identified in (c).

31. Compound prepared by the method of claim 30.
